# EUROPEAN PATENT SPECIFICATION

(11) **EP 1 817 342 B1**
(45) Date of publication and mention of the grant of the patent: **06.03.2013**
(21) Application number: 05817207.3
(22) Date of filing: 02.12.2005
(51) Int. Cl.: C07K 16/06, C07K 1/22

(54) **METHOD FOR AFFINITY PURIFICATION**
VERFAHREN ZUR AFFINITÄTSREINIGUNG
METHODE POUR LA PURIFICATION D'AFFINITÉ

(30) Priority: 02.12.2004 EP 04078282
(43) Date of publication of application: 15.08.2007
(73) Proprietor: BAC IP B.V., 1411 GP Naarden (NL)
(72) Inventor: HERMANS, Wilhelmus, Josephus, Johanna, NL-4751 KR Oud-Gastel (NL); TEN HAAFT, Mark, Ronald, NL-2614 TW Delft (NL); OVERWEEL, Anja, NL-2651 VE Berkel En Rodenrijs (NL)
(74) Representative: Ketelaars, Maarten F.J.M.
(86) International application number: PCT/NL2005/000829
(87) International publication number: WO 2006/059904

(56) References cited:
- WO-A-2004/041862
- WO-A-2004/041867
- MCMAHON M J ET AL: "Polyreactivity as an acquired artefact, rather than a physiologic property, of antibodies: evidence that monoreactive antibodies may gain the ability to bind to multiple antigens after exposure to low pH" JOURNAL OF IMMUNOLOGICAL METHODS, ELSEVIER SCIENCE PUBLISHERS B.V.,AMSTERDAM, NL, vol. 241, no. 1-2, 31 July 2000 (2000-07-31), pages 1-10, XP004213706 ISSN: 0022-1759
- LAMBIN P ET AL: "PURIFICATION OF HUMAN IGG4 SUBCLASS WITH ALLERGEN-SPECIFIC BLOCKING ACTIVITY" JOURNAL OF IMMUNOLOGICAL METHODS, ELSEVIER SCIENCE PUBLISHERS B.V.,AMSTERDAM, NL, vol. 165, no. 1, 1993, pages 99-111, XP009031414 ISSN: 0022-1759
- BIRD P ET AL: "The separation of human serum IgG into subclass fractions by immunoaffinity chromatography and assessment of specific antibody activity." JOURNAL OF IMMUNOLOGICAL METHODS. 8 JUN 1984, vol. 71, no. 1, 8 June 1984 (1984-06-08), pages 97-105, XP002341247 ISSN: 0022-1759
- MONESTIER M ET AL: "RAT MONOCLONAL ANTIBODIES TO MURINE IGM DETERMINANTS APPLICATION TO ANTIBODY PURIFICATION" HYBRIDOMA, vol. 8, no. 6, 1989, pages 631-638, XP009052547 ISSN: 0272-457X
- EWERT STEFAN ET AL: "Biophysical properties of camelid VHH domains compared to those of human VH3 domains" BIOCHEMISTRY, vol. 41, no. 11, 19 March 2002 (2002-03-19), pages 3628-3636, XP002374740 ISSN: 0006-2960
- PÉREZ J M ET AL: "Thermal unfolding of a llama antibody fragment: a two-state reversible process." BIOCHEMISTRY. 9 JAN 2001, vol. 40, no. 1, 9 January 2001 (2001-01-09), pages 74-83, XP002374741 ISSN: 0006-2960
- MUYLDERMANS SERGE ET AL: "Unique single-domain antigen binding fragments derived from naturally occurring camel heavy-chain antibodies" JOURNAL OF MOLECULAR RECOGNITION, vol. 12, no. 2, March 1999 (1999-03), pages 131-140, XP002374742 ISSN: 0952-3499
- VAN DER LINDEN RICHARD ET AL: "Induction of immune responses and molecular cloning of the heavy chain antibody repertoire of Lama glama" JOURNAL OF IMMUNOLOGICAL METHODS, vol. 240, no. 1-2, 23 June 2000 (2000-06-23), pages 185-195, XP002374743 ISSN: 0022-1759
- VERHEESEN P ET AL: "Beneficial properties of single-domain antibody fragments for application in immunoaffinity purification and immuno-perfusion chromatography." BIOCHIMICA ET BIOPHYSICA ACTA, vol. 1624, no. 1-3, 5 December 2003 (2003-12-05), pages 21-28, XP002374744 ISSN: 0006-3002

### SEQUENCE LISTING

<110> Unilever N.V. / BAC Unilever N.V.
<120> Method for affinity purification
<130> P6004885 pct / T7116
<160> 33
<170> PatentIn version 3.3
<210> 1
   <211> 121
   <212> PRT
   <213> Lama glama
<400> 1
   Gln Val Gln Leu Gln Glu Ser Gly Gly Gly Leu Val Gln Pro Gly Gly
   1 5 10 15
   Ser Leu Arg Leu Ser Cys Ala Ala Ser Gly Arg Thr Ile Ser Arg Tyr
   20 25 30
   Ala Met Ser Trp Phe Arg Gln Ala Pro Gly Lys Glu Arg Glu Phe Val
   35 40 45
   Ala Val Ala Arg Arg Ser Gly Asp Gly Ala Phe Tyr Ala Asp Ser Val
   50 55 60
   Gln Gly Arg Phe Thr Val Ser Arg Asp Asp Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ile Asp Ser Asp Thr Phe Tyr Ser Gly Ser Tyr Asp Tyr Trp Gly
   100 105 110
   Gln Gly Thr Gln Val Thr Val Ser Ser
   115 120
<210> 2
   <211> 120
   <212> PRT
   <213> Lama glama
<400> 2
   Gln Val Gln Leu Gln Glu Ser Gly Gly Gly Leu Val Gln Ala Gly Gly
   1 5 10 15
   Ser Leu Arg Leu Ser Cys Glu Ala Thr Gly Leu Thr Phe Ser Gly Val
               20                  25                  30
   Ala Met Ala Trp Phe Arg Gln Ala Pro Gly Lys Glu Arg Glu Phe Val
           35                  40                  45
   Ala Ile Ile Arg Trp Ser Gly Ser Thr Thr Tyr Tyr Ser Asp Ser Ala
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Asp Pro Ser Asp Arg Phe Gly Glu Tyr Asp Tyr Trp Gly Arg
               100                 105                 110
   Gly Thr Gln Val Thr Val Ser Ser
           115                 120
<210> 3
   <211> 121
   <212> PRT
   <213> Lama glama
<400> 3
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Arg Thr Ile Ser Arg Tyr
               20                  25                  30
   Ala Met Ser Trp Phe Arg Gln Ala Pro Gly Lys Glu Arg Glu Phe Val
           35                  40                  45
   Ala Val Ala Arg Arg Ser Gly Asp Gly Ala Phe Tyr Ser Asp Ser Ala
       50                  55                  60
   Glu Gly Arg Phe Thr Val Ser Arg Asp Asp Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ile Asp Ser Ser Thr Asp Tyr Thr Gly Thr Tyr Asp Tyr Trp Gly
   100 105 110
   Gln Gly Thr Gln Val Thr Val Ser Ser
   115 120
<210> 4
   <211> 121
   <212> PRT
   <213> Lama glama
<400> 4
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Thr Ile Ser Arg Tyr
   20 25 30
   Ala Val Ser Trp Phe Arg Gln Ala Pro Gly Lys Glu Arg Glu Phe Val
   35 40 45
   Ala Val Ala Arg Arg Thr Gly Asp Gly Ala Phe Tyr Ser Asp Ser Ala
   50 55 60
   Glu Gly Arg Phe Thr Val Ser Arg Asp Asp Ala Lys Asn Thr Val Tyr
   65 70 75 80
   Leu Gln Met Asn Ser Leu Lys Pro Glu Asp Thr Ala Ile Tyr Tyr Cys
   85 90 95
   Ala Ile Asp Ser Ser Thr Phe Tyr Thr Gly Ser Tyr Asp Tyr Trp Gly
   100 105 110
   Gln Gly Thr Gln Val Thr Val Ser Ser
   115 120
<210> 5
   <211> 121
   <212> PRT
   <213> Lama glama
<400> 5
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Thr Ile Ser Arg Tyr
   20 25 30
   Ala Leu Ser Trp Phe Arg Gln Ala Pro Gly Lys Glu Arg Glu Phe Val
   35 40 45
   Ala Val Ala Arg Arg Ser Gly Asp Gly Ala Phe Tyr Ser Asp Ser Ala
   50 55 60
   Gln Gly Arg Phe Thr Val Ser Arg Asp Asp Ala Lys Asn Thr Val Tyr
   65 70 75 80
   Leu Gln Met Asn Ser Leu Lys Pro Asp Asp Thr Ala Val Tyr Tyr Cys
   85 90 95
   Ala Ile Asp Ser Asp Thr Phe Tyr Ser Gly Ser Tyr Asp Tyr Trp Gly
   100 105 110
   Gln Gly Thr Gln Val Thr Val Ser Ser
   115 120
<210> 6
   <211> 112
   <212> PRT
   <213> Lama glama
<400> 6
   Gln Val Gln Leu Gln Glu Ser Gly Gly Gly Leu Val Gln Ala Gly Gly
   1 5 10 15
   Ser Leu Arg Leu Ser Cys Ala Ala Ser Asp Arg Tyr Phe Ser Ser His
   20 25 30
   Thr Met Ala Trp Phe Arg Gln Ala Pro Gly Lys Glu Arg Glu Phe Val
   35 40 45
   Ala Arg Val Asp Trp Ser Gly Gly Ser Thr Glu Tyr Ala Asp Ser Val
   50 55 60
   Lys Gly Arg Phe Thr Ile Ser Lys Asp Asn Ala Lys Asn Thr Val Tyr
   65 70 75 80
   Leu Gln Met Asp Ser Leu Lys Pro Glu Asp Ala Ala Val Tyr Tyr Cys
   85 90 95
   Ala Ala Gly Pro Tyr Trp Gly Gln Gly Thr Gln Val Thr Val Ser Ser
   100 105 110
<210> 7
   <211> 118
   <212> PRT
   <213> Lama glama
<400> 7
   Gln Val Gln Leu Gln Glu Ser Gly Gly Gly Leu Val Gln Ala Gly Gly
   1               5                   10                  15
   Ser Leu Arg Leu Ser Cys Val Ala Ser Gly Ser Ile Phe Ser Val Asp
               20                  25                  30
   Ile Met Tyr Trp Tyr Arg Gln Ala Pro Gly Lys Gln Arg Glu Arg Val
           35                  40                  45
   Gly Ser Met Tyr Ser Asp Gly Thr Thr Thr Tyr Val Asp Ser Val Lys
       50                  55                  60
   Gly Arg Phe Thr Ile Ser Arg Gly Asp Ser Thr Asp Asn Ala Lys Arg
   65                  70                  75                  80
   Ile Thr Tyr Leu Gln Met Asn Asn Leu Gln Pro Glu Asp Thr Ala Val
                   85                  90                  95
   Tyr Tyr Cys Ala Ala Trp Ala Gly Ser Phe Tyr Trp Gly Ser Gly Thr
               100                 105                 110
   Gln Val Thr Val Ser Ser
           115
<210> 8
   <211> 118
   <212> PRT
   <213> Lama glama
<400> 8
   Gln Val Gln Leu Gln Asp Ser Gly Gly Gly Leu Val Gln Ala Gly Gly
   1               5                   10                  15
   Ser Leu Arg Leu Ser Cys Ala Ala Ser Gly Arg Thr Phe Ser Gly Tyr
               20                  25                  30
   Ser Val Ala Trp Phe Arg Gln Val Pro Gly Lys Glu Arg Glu Leu Val
           35                  40                  45
   Ala Ser Ile Asn Arg Ser Gly Asp Asn Thr Tyr Ile Glu Asp Ser Val
       50                  55                  60
   Lys Gly Arg Phe Thr Ile Ser Thr Asp Asn Thr Lys Ser Thr Ile Tyr
   65 70 75 80
   Leu Gln Leu Asn Ala Leu Lys Pro Glu Asp Thr Ala Val Tyr Tyr Cys
   85 90 95
   Ala Ala Pro Tyr Gly Leu Gly Arg Tyr Asp Ser Trp Gly Gln Gly Thr
   100 105 110
   Gln Val Thr Val Ser Ser
   115
<210> 9
   <211> 118
   <212> PRT
   <213> Lama glama
<400> 9
   Gln Val Gln Leu Gln Glu Ser Gly Gly Gly Leu Val Gln Ala Gly Gly
   1 5 10 15
   Ser Leu Arg Leu Ser Cys Val Ala Ser Gly Ser Ile Phe Ser Val Asp
   20 25 30
   Ile Met Tyr Trp Tyr Arg Gln Ala Pro Gly Lys Gln Arg Glu Arg Val
   35 40 45
   Ala Ser Met Phe Ser Asp Gly Thr Thr Thr Tyr Val Asp Ser Val Lys
   50 55 60
   Gly Arg Phe Thr Ile Ser Arg Gly Asp Ser Thr Asp Asn Ala Lys Arg
   65 70 75 80
   Ile Thr Tyr Leu Gln Met Asn Ser Leu Gln Pro Glu Asp Thr Ala Val
   85 90 95
   Tyr Tyr Cys Ala Ala Trp Ala Gly Ser Phe Tyr Trp Gly Gln Gly Thr
   100 105 110
   Gln Val Thr Val Ser Ser
   115
<210> 10
   <211> 113
   <212> PRT
   <213> Lama glama
<400> 10
   Gln Val Gln Leu Gln Asp Ser Gly Gly Gly Leu Val Gln Ala Gly Gly
   1 5 10 15
   Ser Leu Arg Leu Ser Cys Ala Ala Ser Ser Arg Thr Phe Ser Val Tyr
   20 25 30
   Thr Val Ala Trp Phe Arg Gln Thr Pro Gly Gly Lys Glu Arg Glu Phe
   35 40 45
   Val Ala Gln Ile Asn Trp Asn Gly Asp Ser Thr Asn Tyr Ala Asp Ser
   50 55 60
   Val Lys Gly Arg Phe Thr Val Ser Arg Asp Asn Val Lys Asn Thr Val
   65 70 75 80
   Phe Leu Gln Met Ser Thr Leu Lys Pro Glu Asp Thr Ala Val Tyr Tyr
   85 90 95
   Cys Ala Ala Gly Ser Asn Trp Gly Gln Gly Thr Gln Val Thr Val Ser
   100 105 110
   Ser
<210> 11
   <211> 112
   <212> PRT
   <213> Lama glama
<400> 11
   Gln Val Gln Leu Gln Asp Ser Gly Gly Gly Leu Val Gln Ala Gly Gly
   1 5 10 15
   Ser Leu Arg Leu Ser Cys Ala Ala Ser Gly Arg Ile Phe Ser Thr His
   20 25 30
   Thr Met Ala Trp Phe Arg Gln Ala Pro Gly Gln Glu Arg Glu Phe Val
   35 40 45
   Ser Ser Ile Ser Trp Asn Gly Ala Val Thr Asn Tyr Leu Asp Ser Val
   50 55 60
   Lys Gly Arg Phe Thr Ile Ser Lys Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Gly Arg Asn Trp Gly Gln Gly Thr Gln Val Thr Val Ser Ser
   100 105 110
<210> 12
   <211> 121
   <212> PRT
   <213> Lama glama
<400> 12
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Thr Phe Arg Asn Tyr
   20 25 30
   Ala Met Ala Trp Phe Arg Gln Ala Pro Gly Lys Glu Arg Glu Phe Val
   35 40 45
   Ala Val Ile Arg Arg Gly Gly Gly Asp Thr Tyr Tyr Ser Asp Ser Ala
   50 55 60
   Glu Gly Arg Phe Thr Ile Ser Arg Asp Asn Ala Lys Asn Thr Val Tyr
   65 70 75 80
   Leu Gln Met Asn Asn Leu Lys Pro Glu Asp Thr Ala Val Tyr Tyr Cys
   85 90 95
   Ala Ala Asp Val Ser Asp Asp Asn Thr Gly Lys Tyr Asp Tyr Trp Gly
   100 105 110
   Gln Gly Thr Gln Val Thr Val Ser Ser
   115 120
<210> 13
   <211> 126
   <212> PRT
   <213> Lama glama
<400> 13
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Thr Ser Ser Ile His
   20 25 30
   Leu Met Ala Trp Phe Arg Gln Ala Pro Gly Lys Glu Arg Glu Phe Leu
   35 40 45
   Ala Ser Ile Ile Trp Thr Gly Gly Thr Thr Tyr Tyr Ala Gly Thr Thr
   50 55 60
   Phe Tyr Gly Asp Ser Ala Glu Gly Arg Phe Thr Ile Ser Asn Asp Asn
   65 70 75 80
   Ala Arg Asn Thr Gly Tyr Leu Gln Met Asn Ser Leu Lys Pro Glu Asp
   85 90 95
   Thr Ala Val Tyr Tyr Cys Ala Ala Thr Ser Leu Val Asn Pro Thr Arg
   100 105 110
   Tyr Asp Tyr Trp Gly Gln Gly Thr Gln Val Thr Val Ser Ser
   115 120 125
<210> 14
   <211> 126
   <212> PRT
   <213> Lama glama
<400> 14
   Gln Val Gln Leu Gln Glu Ser Gly Gly Gly Leu Val Gln Ala Gly Gly
   1 5 10 15
   Ser Leu Arg Leu Ser Cys Ala Ala Ser Gly Arg Thr Ser Ser Ile His
   20 25 30
   Leu Met Ala Trp Phe Arg Gln Ala Pro Gly Lys Glu Arg Glu Phe Val
   35 40 45
   Ala Ser Ile Ile Trp Thr Gly Gly Thr Thr Tyr Tyr Ala Gly Thr Thr
   50 55 60
   Phe Tyr Gly Asp Ser Ala Glu Gly Arg Phe Thr Ile Ser Asn Asp Asn
   65 70 75 80
   Ala Arg Asn Thr Gly Tyr Leu Gln Met Asn Ser Leu Lys Pro Glu Asp
   85 90 95
   Thr Ala Val Tyr Tyr Cys Ala Ala Thr Ser Leu Glu Asn Pro Thr Arg
   100 105 110
   Tyr Asp Tyr Trp Gly Gln Gly Thr Gln Val Thr Val Ser Ser
   115 120 125
<210> 15
   <211> 126
   <212> PRT
   <213> Lama glama
<400> 15
   Gln Val Gln Leu Gln Glu Ser Gly Gly Gly Leu Val Gln Ala Gly Gly
   1               5                   10                  15
   Ser Leu Arg Leu Ser Cys Thr Ala Ser Gly Arg Thr Ser Ser Ile His
               20                  25                  30
   Leu Met Ala Trp Phe Arg Gln Ala Pro Gly Lys Glu Arg Glu Phe Val
           35                  40                  45
   Ala Ser Ile Ile Trp Thr Gly Gly Thr Thr Tyr Tyr Ala Gly Thr Thr
       50                  55                  60
   Phe Tyr Gly Asp Ser Ala Glu Gly Arg Phe Thr Ile Ser Asn Asp Asn
   65                  70                  75                  80
   Ala Arg Asn Thr Gly Tyr Leu Gln Met Asn Ser Leu Lys Pro Glu Asp
                   85                  90                  95
   Thr Ala Val Tyr Tyr Cys Ala Ala Thr Ser Leu Glu Asn Pro Thr Arg
               100                 105                 110
   Tyr Asp Tyr Trp Gly Gln Gly Thr Gln Val Thr Val Ser Ser
           115                 120                 125
<210> 16
   <211> 128
   <212> PRT
   <213> Lama glama
<400> 16
   Gln Val Gln Leu Gln Asp Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asp Gly Ser Ser Asp Gly Tyr Thr Tyr Tyr
   50                  55                  60
   Ala Asp Ser Val Lys Gly Arg Phe Thr Ile Ser Arg Asp Asn Ala Lys
   65                  70                  75                  80
   Asn Thr Val Tyr Leu Gln Met Asn Asn Leu Lys Pro Glu Asp Thr Ala
                   85                  90                  95
   Val Tyr Tyr Cys Ala Ala Arg Leu Phe Gly Val Ala Thr Thr Asp Pro
               100                 105                 110
   Ser Tyr Phe Asp Ser Trp Gly Gln Gly Thr Gln Val Thr Val Ser Ser
           115                 120                 125
<210> 17
   <211> 126
   <212> PRT
   <213> Lama glama
<400> 17
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Leu Asp Tyr Tyr
               20                  25                  30
   Ala Ile Gly Trp Phe Arg Gln Ala Pro Gly Lys Glu Arg Glu Gly Ile
           35                  40                  45
   Ser Cys Ile Ser Ser Ser Asp Ser Asp Thr Tyr Tyr Glu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Arg His Tyr Gly Leu Cys Val Val Asp Arg Ala Tyr Tyr Ala
               100                 105                 110
   Met Gly Tyr Trp Gly Lys Gly Thr Pro Val Thr Val Ser Ser
           115                 120                 125
<210> 18
   <211> 128
   <212> PRT
   <213> Lama glama
<400> 18
   Gln Val Gln Leu Gln Asp Ser Gly Gly Gly Leu Val Gln Pro Gly Gly
   1               5                   10                  15
   Ser Leu Arg Leu Ser Cys Ala Ala Ser Gly Ser Thr Leu Asp Tyr Tyr
               20                  25                  30
   Ala Ile Gly Trp Phe Arg Gln Ala Pro Gly Lys Glu Arg Glu Gly Val
           35                  40                  45
   Ser Cys Met Ser Ser Ser Asp Gly Ser Ser Asp Gly Tyr Thr Tyr Tyr
       50                  55                  60
   Glu Asp Ser Val Lys Gly Arg Phe Thr Ile Ser Arg Asp Asn Ala Lys
   65                  70                  75                  80
   Asn Thr Val Tyr Leu Gln Met Asn Asn Leu Lys Pro Glu Asp Thr Ala
                   85                  90                  95
   Val Tyr Tyr Cys Ala Ala Arg Leu Tyr Gly Ile Ala Thr Thr Asp Pro
               100                 105                 110
   Ser Tyr Phe Asp Ser Trp Gly Gln Gly Thr Gln Val Thr Val Ser Ser
           115                 120                 125
<210> 19
   <211> 124
   <212> PRT
   <213> Lama glama
<400> 19
   Gln Val Gln Leu Gln Asp Ser Gly Gly Gly Leu Val Gln Pro Gly Gly
   1               5                   10                  15
   Ser Leu Arg Leu Ser Cys Ala Ala Ser Gly Val Thr Leu Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asn Gly Tyr Thr Tyr Tyr Glu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
   85 90 95
   Ala Ala Arg Leu Phe Gly Val Ala Thr Met Asp Pro Ser Tyr Phe Gly
   100 105 110
   Ser Trp Gly Gln Gly Thr Gln Val Thr Val Ser Ser
   115 120
<210> 20
   <211> 126
   <212> PRT
   <213> Lama glama
<400> 20
   Gln Val Gln Leu Gln Asp Ser Gly Gly Ala Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Arg Glu Arg Glu Gly Ile
   35 40 45
   Ser Cys Ile Ser Ser Asn Val Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Arg His Tyr Gly Leu Cys Val Val Asp Arg Tyr Tyr Tyr Asp
   100 105 110
   Met Gly Tyr Trp Gly Lys Gly Thr Pro Val Thr Val Ser Ser
   115 120 125
<210> 21
   <211> 130
   <212> PRT
   <213> Lama glama
<400> 21
   Gln Val Gln Leu Gln Glu Ser Gly Gly Gly Leu Val Gln Pro Gly Gly
   1 5 10 15
   Ser Leu Arg Leu Ser Cys Ala Ala Ser Gly Phe Thr Leu Asp Tyr Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Ser Asp Gly Tyr Thr Tyr Tyr
   50 55 60
   Ala Asp Ser Val Lys Gly Arg Phe Thr Ile Ser Arg Asp Asn Ala Lys
   65 70 75 80
   Asn Thr Val Tyr Leu Gln Met Asn Ser Leu Lys Pro Glu Asp Met Ala
   85 90 95
   Val Tyr Tyr Cys Ala Ala Arg His Tyr Gly Leu Cys Val Val Asp Pro
   100 105 110
   Asp Tyr Tyr Gly Lys Gly Tyr Trp Gly Lys Gly Thr Pro Val Thr Val
   115 120 125
   Ser Ser
   130
<210> 22
   <211> 124
   <212> PRT
   <213> Lama glama
<400> 22
   Gln Val Gln Leu Gln Asp Ser Gly Gly Gly Leu Val Gln Pro Gly Gly
   1 5 10 15
   Ser Leu Arg Leu Ser Cys Ala Ala Ser Gly Phe Thr Leu Asp Asn Tyr
   20 25 30
   Ala Ile Gly Trp Phe Arg Gln Ala Pro Gly Lys Glu Arg Glu Gly Val
   35 40 45
   Ser Cys Ile Ser Ser Gly Asn Ser Tyr Thr Tyr Tyr Glu Asp Pro Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Arg Leu Phe Gly Val Ala Thr Met Asp Pro Ser Tyr Phe Asp
   100 105 110
   Ser Trp Gly Gln Gly Thr Gln Val Thr Val Ser Ser
   115 120
<210> 23
   <211> 119
   <212> PRT
   <213> Lama glama
<400> 23
   Gln Val Gln Leu Gln Glu Ser Gly Gly Gly Leu Val Gln Pro Gly Gly
   1 5 10 15
   Ser Leu Arg Leu Ser Cys Ala Ala Ser Gly Ser Ile Phe Ser Ile Asn
   20 25 30
   Phe Ala Ala Trp Tyr Arg Gln Ala Pro Gly Gln Gln Arg Glu Trp Val
   35 40 45
   Ala Ile Val Thr Ser Ser Gly Thr Thr Asn Tyr Ala Asp Ser Val Lys
   50 55 60
   Gly Arg Phe Thr Ile Ser Arg Asp Tyr Ala Lys Lys Thr Val Tyr Leu
   65 70 75 80
   Gln Met Asn Ser Leu Ile Pro Glu Asp Thr Gly Val Tyr Tyr Cys Asn
   85 90 95
   Gly Asn Phe Gly Thr Ala Leu Thr Asn Leu Ser Ala Trp Gly Gln Gly
   100 105 110
   Thr Gln Val Thr Val Thr Ser
   115
<210> 24
   <211> 120
   <212> PRT
   <213> Lama glama
<400> 24
   Gln Val Gln Leu Gln Asp Ser Gly Gly Gly Leu Val Gln Ala Gly Gly
   1 5 10 15
   Ser Leu Arg Leu Ser Cys Ala Ala Ser Gly Ser Ile Phe Ser Ile Asn
               20                  25                  30
   Val Ala Tyr Trp Tyr Arg Gln Ala Pro Gly Lys Gln Arg Glu Trp Val
           35                  40                  45
   Ala Gly Ile Met Ser Gly Gly Thr Thr Asn Tyr Ala Asp Ser Val Lys
       50                  55                  60
   Gly Arg Phe Thr Ile Ser Arg Asp Asn Asp Lys Asn Met Val Tyr Leu
   65                  70                  75                  80
   Gln Met Asn Ser Leu Thr Pro Ala Asp Thr Ala Val Tyr Tyr Cys Asn
                   85                  90                  95
   Ala Asn Val Arg Gln Lys Ser Gly His Asn Ile Ser Ala Trp Gly Gln
               100                 105                 110
   Gly Thr Gln Val Thr Val Ser Ser
           115                 120
<210> 25
   <211> 120
   <212> PRT
   <213> Lama glama
<400> 25
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Ser Ile Phe Ser Ile Asn
               20                  25                  30
   Val Ala Tyr Trp Tyr Arg Gln Ala Pro Gly Lys Gln Arg Glu Trp Val
           35                  40                  45
   Ala Gly Ile Met Ser Gly Gly Thr Thr Asn Tyr Ala Asp Ser Val Lys
       50                  55                  60
   Gly Arg Phe Thr Ile Ser Arg Asp Asn Asp Lys Asn Met Val Tyr Leu
   65                  70                  75                  80
   Gln Met Asn Ser Leu Thr Pro Ala Asp Thr Ala Val Tyr Tyr Cys Asn
                   85                  90                  95
   Ala Asn Val Arg Gln Asn Ser Gly His Asn Ile Ser Ala Trp Gly Gln
               100                 105                 110
   Gly Thr Gln Val Thr Val Ser Ser
           115                 120
<210> 26
   <211> 119
   <212> PRT
   <213> Lama glama
<400> 26
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Ser Ile Phe Ser Ile Asn
               20                  25                  30
   Val Met Ala Trp Tyr Arg Gln Ala Pro Gly Gln Gln Arg Glu Trp Val
           35                  40                  45
   Ala Ile Val Thr Ser Ser Gly Thr Thr Asn Tyr Ala Asp Ser Val Lys
       50                  55                  60
   Gly Arg Phe Thr Ile Ser Arg Asp Tyr Ala Lys Lys Thr Val Tyr Leu
   65                  70                  75                  80
   Gln Met Asn Ser Leu Lys Pro Glu Asp Thr Ala Val Tyr Asn Cys Asp
                   85                  90                  95
   Ala Asn Ile Asn Ser Arg Val Gly Arg Ile Ser Ala Arg Gly Gln Gly
               100                 105                 110
   Thr Gln Val Thr Val Ser Ser
           115
<210> 27
   <211> 124
   <212> PRT
   <213> Lama glama
<400> 27
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Val Thr Leu Asp Asp Tyr
               20                  25                  30
   Ala Ile Gly Trp Phe Arg Gln Ala Pro Gly Lys Glu Arg Glu Gly Val
           35                  40                  45
   Ser Cys Ile Ser Ser Ser Asn Gly Tyr Thr Tyr Tyr Glu Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Ser Leu Lys Pro Glu Asp Thr Ala Val Tyr Tyr Cys
                   85                  90                  95
   Ala Ala Ser Leu Phe Gly Val Ala Thr Met Asp Pro Ser Tyr Phe Gly
               100                 105                 110
   Ser Trp Gly Arg Gly Thr Gln Val Thr Val Ser Ser
           115                 120
<210> 28
   <211> 123
   <212> PRT
   <213> Lama glama
<400> 28
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Phe Thr Phe Asp Asp Tyr
               20                  25                  30
   Ala Ile Gly Trp Phe Arg Gln Ala Pro Gly Lys Glu Arg Glu Gly Ile
           35                  40                  45
   Ser Cys Val Ser Ser Ser Asp Asp Thr Tyr Tyr Ala Asp Ser Val Lys
       50                  55                  60
   Gly Arg Phe Thr Ile Ser Ser Asp Asn Ala Lys Asn Thr Met Tyr Leu
   65                  70                  75                  80
   Gln Met Asn Ser Leu Lys Pro Glu Asp Thr Ala Val Tyr Tyr Cys Ala
                   85                  90                  95
   Ala Arg Leu Trp Gly Leu Cys Ala Val Asp Glu Ala Tyr Phe Thr Ser
               100                 105                 110
   Trp Gly Gln Gly Thr Gln Val Thr Val Ser Ser
   115 120
<210> 29
   <211> 123
   <212> PRT
   <213> Lama glama
<400> 29
   Gln Val Gln Leu Gln Glu Ser Gly Gly Gly Leu Val Ala Gly Gly Ser
   1 5 10 15
   Leu Arg Leu Ser Cys Ala Ala Ser Gly Phe Thr Phe Asp Asp Tyr Ala
   20 25 30
   Ile Gly Trp Phe Arg Gln Ala Pro Gly Lys Glu Arg Glu Gly Val Ser
   35 40 45
   Cys Ile Ser Ser Ser Asp Gly His Thr Tyr Ser Val Asp Ser Val Lys
   50 55 60
   Gly Arg Phe Thr Ile Ser Arg Asp Asn Ala Lys Asn Thr Val Tyr Leu
   65 70 75 80
   Gln Met Asn Ser Leu Gln Pro Glu Asp Thr Ala Val Tyr Tyr Cys Ala
   85 90 95
   Ala Arg Arg Trp Gly Leu Cys Thr Val Asp Val Pro Tyr Phe His Ser
   100 105 110
   Trp Gly Gln Gly Thr Gln Val Thr Val Ser Ser
   115 120
<210> 30
   <211> 128
   <212> PRT
   <213> Lama glama
<400> 30
   Gln Val Gln Leu Gln Asp Ser Gly Gly Gly Leu Val Gln Pro Gly Gly
   1 5 10 15
   Ser Leu Arg Leu Ser Cys Ala Ala Ser Gly Phe Thr Leu Lys Asp Tyr
   20 25 30
   Ala Ile Gly Trp Phe Arg Gln Ala Pro Gly Lys Glu Arg Glu Gly Val
   35 40 45
   Ser Cys Ile Ser Ser Ser Asp Gly Ser Ser Asp Gly Trp Thr Tyr Tyr
   50 55 60
   Ala Asp Ser Val Lys Gly Arg Phe Thr Ile Ser Arg Asp Asn Ala Lys
   65 70 75 80
   Asn Thr Val Tyr Leu Gln Met Asn Asn Leu Lys Pro Glu Asp Thr Ala
   85 90 95
   Val Tyr Tyr Cys Ala Ala Arg Leu Phe Gly Val Ala Thr Thr Asp Pro
   100 105 110
   Ser Tyr Phe Asp Ser Trp Gly Gln Gly Thr Gln Val Thr Val Ser Ser
   115 120 125
<210> 31
   <211> 126
   <212> PRT
   <213> Lama glama
<400> 31
   Gln Val Gln Leu Gln Glu Ser Gly Gly Ala Leu Val Gln Pro Gly Gly
   1 5 10 15
   Ser Leu Arg Leu Ser Cys Ala Ala Ser Gly Phe Thr Leu Asp Asp Tyr
   20 25 30
   Ala Ile Gly Trp Phe Arg Gln Ala Pro Gly Arg Glu Arg Glu Gly Ile
   35 40 45
   Ser Cys Ile Ser Ser Asn Val Gly Ser Thr Tyr Tyr Ala Asp Ser Val
   50 55 60
   Lys Gly Arg Phe Thr Ile Ser Arg Asp Asn Ala Lys Asn Thr Val Tyr
   65 70 75 80
   Leu Gln Met Asn Ser Leu Lys Pro Glu Asp Thr Ala Val Tyr Tyr Cys
   85 90 95
   Ala Ala Arg Gln Tyr Gly Leu Cys Val Val Asp Arg Tyr Tyr Tyr Asp
   100 105 110
   Met Gly Tyr Trp Gly Lys Gly Thr Pro Val Thr Val Ser Ser
   115 120 125
<210> 32
   <211> 124
   <212> PRT
   <213> Lama glama
<400> 32
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Leu Asp Asp Tyr
               20                  25                  30
   Ala Ile Gly Trp Phe Arg Gln Ala Pro Gly Lys Glu Arg Glu Gly Phe
           35                  40                  45
   Ser Cys Ile Ser Ser Ser Ala Gly Asn Thr Tyr Tyr Glu Asp Ser Val
       50                  55                  60
   Lys Gly Arg Phe Thr Ile Ser Arg Asp Lys Ala Lys Asn Thr Ala Tyr
   65                  70                  75                  80
   Leu Gln Met Asn Ser Leu Lys Pro Glu Asp Thr Ala Val Tyr Tyr Cys
                   85                  90                  95
   Ala Thr Arg Leu Phe Gly Ile Cys Thr Val Asp Ala Gly Tyr Phe Gly
               100                 105                 110
   Ser Trp Gly Gln Gly Thr Gln Val Thr Val Ser Ser
           115                 120
<210> 33
   <211> 121
   <212> PRT
   <213> Lama glama
<400> 33
   Gln Val Gln Leu Gln Glu Ser Gly Gly Gly Leu Val Gln Ala Leu Val
   1               5                   10                  15
   Ser Leu Lys Leu Ser Cys Ala Ala Ser Gly Leu Pro Ser Gly Ser Tyr
               20                  25                  30
   Asp Met Gly Trp Phe Arg Gln Arg Pro Gly Lys Glu Arg Glu Phe Val
           35                  40                  45
   Ala Phe Ile Ser Gly Asp Gly Gly Ile Ile Glu Tyr Ser Gly Ser Val
       50                  55                  60
   Lys Gly Arg Phe Thr Ile Ser Arg Asp Asn Ala Lys Asn Thr Met Tyr
   65 70 75 80
   Leu Gln Met Asn Ser Leu Lys Pro Glu Asp Thr Ala Val Tyr Ser Cys
   85 90 95
   Ala Ala Tyr Arg Leu Thr Ala Asp Ala Tyr Asn Phe Arg Tyr Trp Gly
   100 105 110
   Gln Gly Thr Gln Val Thr Val Ser Ser
   115 120

## Description

### Field of the invention

The invention relates to a method for affinity purification and to a binding agent for use in that process.

### Background of the invention

The use of binding agents in affinity purification is known from EP-A-434317. This document discloses that it is possible to immobilise small specific binding agents on a solid phase carrier. The binding agents are especially composed of corresponding VH and VL domain proteins, held together by their natural interaction. On immobilization these small binding agents maintain affinity for their target ligand. An example of the technology disclosed is the immobilisation of an anti-lysozyme Fv on agarose and its use as an immunoadsorbent. The immunoadsorbent comprising immobilized anti-lysozyme-Fv was packed in a column and on this column a mixture of lysozyme and other protein was loaded. The column was washed to remove non-bound protein and subsequently bound protein was eluted.

In this type of immunoaffinity systems the binding strength, specificity and capacity of the immunoadsorbent are important parameters.

Binding strength and specificity refer to the binding between the binding agent and the target. Especially the binding agent is an antibody or fragment thereof, and the binding is highly specific for the target. Desirably, specific binding to the target is maximized and non-specific binding is minimised. Binding with only very limited specificity is underlying the commercial purification systems that are based on protein A and protein L. These are known to bind to a wide range of immunoglobulin molecules such as immunoglobulins from human, mouse and rat.

The strength of the binding should be balanced between good binding such that the column is easily loaded with target material and no target material is eluted prior to elution, and the wish to use mild elution conditions to avoid unneccessary harm to the target molecules. Very stringent elution conditions are undesired because they may lead to denaturation, fragmentation or other defects of the target material. Furthermore mild conditions are benefical to the column material and increase the lifetime thereof.

Capacity of the immunoadsorbent indicates the amount of target material that can be bound by the immunoadsorbent material under binding conditions. Once maximum capacity of the immunoadsorbent material has been obtained, the further loading of target will not result in binding but will cause leakage of target material in the eluent. This either results in a loss of yield in protein purification or requires the inclusion of (repeated) further process steps.

In EP-A-434317 some of these objectives are addressed for example by use of variable domains having reduced affinity for antigen, leading to elution or desorption under milder conditions. Non-specific binding is reduced by reducing the size of the I binding agent down to the minimum required for binding.

McNahon et al. (2000, Journal of Immunological methods 241:1-10) disclose a murine monoclonal IgM anti-goat IgG designated M11. The authors describe that this monoclonal was monoreactive before purification, whereas it was polyreactive to many different proteins after purification.

Lambin et al. (1993, Journal of Immunological methods 165:99-111) disclose a method for purifying to a great degree an IgG4 fraction containing predominantly anti-pollen antibody with blocking activity on an immuno-affinity column comprising human monoclonal antibodies.

Bird et al. (1984, Journal of Immunological methods 71:97-105) disclose the use of human subclass-specific (anti-IgG1; anti-IgG2; anti-IgG3; anti-IgG4 specific) and human subclass-restricted (anti-IgG2, 3 and 4, non-IgG1; anti-IgG1, 2 and 4, non-IgG3; anti-IgG1, 2 and 3, non IgG4) murine monoclonal antibodies to standardise immunoaffinity procedures to allow the isolation of pure polyclonal subclass preparations.

Monestier et al. (1989, Hybridoma 8(6):631-638) disclose a method for the purification of murine IgM antibodies. The authors disclose immunization of rats with a murine monoclonal IgM-kappa antibody (TEPC 183) and three weeks later with a murine monoclonal IgM-lambda antibody (MOPC 104E), to generate rat monoclonal antibodies to murine IgM. Two rat monoclonal antibodies were obtained that bind to both TEPC 183 and MOPC 104E. Binding was observed to be independent of the light chain.

WO 2004/041862 discloses anti-tumour necrosis factor-alpha (TNF-alpha) polypeptides comprising one or more single domain antibodies directed against TNF-alpha. In addition, their use in diagnosis and therapy has been disclosed.

WO 2004/041867 discloses a method of administering protein therapeutic molecules via different administration routes so as to avoid inactivation. The application discloses a polypeptide construct comprising at least one single domain antibody directed against the constant region of IgE.

Ewert et al. (2002, Biochemistry 41:3628-3636) present biophysical properties of camelid VHH domains compared to the properties of human VH3 domains. The authors conclude that the camelid VHH fragments are characterized by a favorable stability and by their ability to reversibly melt without aggregation, thereby allowing the fragments to regain binding affinity after heat renaturation.

Pérez et al. (2001, Biochemistry 40:74-83) investigate the thermostability of heavy chain antibodies from camelids.

Muyldermans and Lauwereys (1999, Journal of Molecular Recognition 12:131-140) present a review of camelid heavy chain-only antibodies. The authors discuss the single chain only antibody versus traditional antibodies and indicate that they expect that camelid single domain antibodies will be applied into a number of biotechnological or medical applications.

Van der Linden et al. (2000, Journal of Immunological methods 240:185-195) address the question whether llamas are a practical source of antigen-specific VHH fragments and concluded that antigen-specific VHH antibodiy fragments are readily accessible from the llama antibody raised against human chorionic gonadotropin.

Although these measures to some extent may improve the capacity, specificity and affinity characteristics of the immunoadsorbent, there is still a desire for further improved immunoadsorbent materials.

### Summary of the invention

We have surprisingly found that an immunoadsorbent material comprising a specific binding agent, which is preferably an antibody or fragment thereof, that binds to at least two epitopes on a target, has the desired high affinity while elution can still be done under mild conditions. When used as a material for column chromatography it was found to lead to high capacity of the column.
Therefore the invention relates to a method for purification of an immunoglobulin, wherein:
a) the method comprises the step of binding the immunoglobulin to an immunoadsorbent material that comprises at least one mono-specific binding agent;
b) the binding agent has affinity for at least two epitopes on the immunoglobulin that are spatially separated by at least 30 angstrom; and,
c) the mono-specific binding agent is a variable domain of an antibody obtainable from a camelid that consists of only heavy chains and that is naturally devoid of light chains.

In another aspect the invention relates to a method for purification of a target molecule by immunoaffinity, wherein the target molecule is an immunoglobulin, which method comprises use of an immunoadsorbent material comprising at least two different binding agents, wherein the binding agents comprise a binding agent with binding affinity for an epitope (one) on the target molecule and a binding agent with binding affinity for a different epitope (two) on the target molecule.

### Description of the invention

Target molecule is defined as a molecule that should bind to a binding agent such as an antibody. Examples of target molecules are proteins that require purification, proteins that are to be identified.

Binding affinity (KD)is defined in the context of specific binding and is represented by an affinity of at least 1*10⁶ M⁻¹. Preferably the binding affinity (measured on the isolated binding agent and target), is at least 1*10⁷ M⁻¹' more preferred at least 1*10⁸ M⁻¹.

Immunoadsorbent material is herein understood to mean the combination of a carrier and a binding agent that is immobilized on the carrier. The carrier may be any material that may be used to for immobilization of a binding agent. Suitable examples are matrix materials, to entrap the binding agent, cell surfaces on which the binding agent is displayed and polymers that can be covalently linked to the binding agent. The person skilled in the art of affinity chromatography is well aware of suitable carriers such as e.g. Sepharose. The binding agent is preferably immobilized onto the carrier by a covalent link.

An epitope is defined as the portion of the target molecule that is bound by the binding agent. If the binding agent is an antibody, it is the portion of a target molecule that triggers an immunological response on immunization of a species with this molecule. Generally it is the site of the target molecule where binding to an antibody takes place.

The epitope is preferably present naturally in the target molecule. Optionally the epitope(s) is/are a sequence that has been artificially included in the target molecule. Optionally a multitude of the same or different epitopes is included in the target molecule to facilitate its purification and detection.

The binding agent is a component that specifically binds to the target molecule with the desired binding affinity. The binding agent is a mono-specific binding agent. A composition comprising a mono-specific binding agent, such as the immunoadsorbant materials of the present invention, is understood to mean a composition having a homogeneous population of the binding agent. It follows that the mono-specific binding agent is specific for a single epitope or ligand. It is however expressly included in the invention that the immunoadsorbant material may comprises more than one type of mono-specific binding agent, each consisting of a homogeneous population. Usually, however, in the context of the present invention, an immunoadsorbant material will not comprise more than 4, 6, 8, 10 or 20 different mono-specific binding agents. It is highly preferred that the binding agent is an antibody or fragment thereof. In that case the mono-specific binding agent will thus be a monoclonal antibody or a fragment thereof, which may be obtained from a hybridoma or expressed from a cloned coding sequence. The term mono-specific binding agent as used herein thus excludes polyclonal antibodies and antisera. An examples of a suitable fragment that may be used is the binding part of the antibody, referred to as CDR, (complementary determining region). Such fragments may suitably be inserted on a natural or synthetic scaffold molecule such as a synthetic peptide. The invention relates to a method of immunoaffinity purification, which comprises the use of a binding agent, which binds to a target molecule in at least two positions. This is referred to as multivalent binding. In one embodiment the binding agent binds to an epitope that is present at least twice on the target molecule. In another embodiment the method uses at least two different binding agents, each binding to different epitopes on the target molecule.

Accordingly the invention relates to a method for purification of a target molecule by immunoaffinity, which comprises use of an immunoadsorbent material comprising a binding agent, which comprises either
a) A binding agent with binding affinity for an epitope that is present at least twice in the target molecule
b) or comprise one binding agent with binding affinity for an epitope (one) on the target molecule and a different binding agent with binding affinity for a different epitope (two) on the target molecule.
Below preferred further steps of this method are presented.

In an alternative embodiment, the invention relates to a method for purification of a target molecule by immunoaffinity which comprises the use of a binding agent according to (a) or (b) above, in combination with at least one binding agent that shows monovalent binding to the target molecule. Such multiple binding agent combinations are especially suitable for use if the individual binding agent has a binding affinity below 1*10⁸ M⁻¹, for the target.

Optionally the method of the invention is a combination of (a) and (b) above.

In a preferred embodiment the invention relates to a method for purification of a target molecule, wherein the method comprises the step of binding the target molecule to an immunoadsorbent material that comprises at least one mono-specific binding agent, and wherein the binding agent has affinity for at least two epitopes on the target molecule that are spatially separated.

Preferably, the spatial separation of the at least two epitopes on the target molecule is such that binding of a first epitope on the target molecule to a binding agent does not sutstantially interfere with binding of a second or further epitope to a binding agent. This means that binding of a first epitope on the target molecule to a binding agent does not substantially reduce or "block" binding of a second or further epitope to a binding agent. The absence of substantial blocking is herein understood to mean that the binding of a first epitope on the target molecule to a binding agent does not reduce the binding of a second or further epitope to a binding agent by more than 50, 40, 30, 20, 10 or 5%. The absence of substantial blocking may be determined in standard cross-blocking assays (see e.g. "Using Antibodies" E. Harlow and D. Lane, Cold Spring Harbor Laboratory Press NY, 1999) or as described in Example 5 herein.

The spatial separation of the at least two epitopes on the target molecule is thus preferably such that at least two epitopes on the target molecule are available for binding to a binding agent on the immunoadsorbent material. The spatial separation of the at least two epitopes is thus such that it results in multivalent binding. The multivalent binding results in a significantly lower dissociation rate (k-diss) of the target molecule for the immunoadsorbent material comprising the binding agent(s) and significantly higher K_{D} values. Preferably the presence of at least two spatially separated epitopes on the target molecule produces at least a 5-, 10-, 50-, 100-, 500- or 1000-fold increase in affinity or K_{D} value for the immunoadsorbant material comprising a binding agent as compared to the affinity or K_{D} value of a target molecule comprising only a single copy of the same epitope. It is herein understood that 120 fM is a higher affinity or K_{D} value than e.g. 6.3 nM. The presence of at least two spatially separated epitopes on the target molecule produces thus induces avidity for the immunoadsorbant material comprising a binding agent as compared to a target molecule comprising only a single copy of the epitope. Avidity is herein understood to refer to the strength of binding of a target molecule with multiple binding sites by a larger complex of binding agents, i.e. the strength of binding of multivalent binding. Affinity, on the other hand refers to simple monovalent receptor ligand systems.

Thus in a preferred embodiment, the avidity of the immunoadsorbant material for the target molecule is at least 5-, 10-, 50-, 100-, 500- or 1000-fold higher than the lowest affinity of a binding agent for an individual epitope on the target molecule. The lowest affinity herein refers to the situation wherein the immunoadsorbant material comprises more than one type of binding agent for immunologically distinct epitopes on the target molecule and wherein each individual type of binding agent may have a different affinity for its epitope. In the situation wherein a single type of binding agent binds an epitope that is repeated on the target molecule the lowest affinity is the affinity of the binding agent for a test molecule comprising only a single copy of the epitope.

The spatial separation of the at least two epitopes on the target molecule is further thus preferably such that the dynamic binding capacity of the immunoadsorbant material comprising a binding agent for the target molecule is increase by at least 10, 20, 50, 100 or 200% as compared to the dynamic binding capacity of the material for to a target molecule comprising only a single copy of the epitope. The dynamic binding capacity (DBC) is herein defined as the elution peak area devised by the total peak area (flowthrough + elution) and this multiplied with the amount of target molecule (see Example 3).

Another important feature of the current invention is that although multivalent binding of the target molecule to the immunoadsorbant material leads to high binding affinities or avidity for the target molecule, the release of said molecules can still be obtained at mild elution conditions. Thus in a preferred embodiment, the avidity of an immunoadsorbant material comprising a binding agent for the target molecule is at least 1*10⁹ M⁻¹, 1*10¹⁰ M⁻¹, 1*10¹¹ M⁻¹, or 1*10¹² M⁻¹. Preferably, at the same at least 90% of the target molecule can be eluted from the immunoadsorbant material at a pH of 2.5, 2.75, 3.0, 3.25 3.5 or higher. However, preferably the individual affinities of a binding agent for the an epitope on the target molecule are less than 1*10¹⁰ M⁻¹, 1*10¹¹ M⁻¹, or 1*10¹² M⁻¹.

The above defined features of binding of the target molecule to the immunoadsorbant material may be achieved when the at least two epitopes on the target molecule are spatially separated by at least 10, 20, 25, 30,40, 50 or 60 Ǻngstrom.

In the methods of the invention, the at least two epitopes on the target molecule may be at least two immunologically distinct epitopes. In which case at least two distinct binding agents are required in the immunoadsorbant material, preferably one for each immunologically distinct epitope.

Alternatively, the methods of the invention, the at least two epitopes on the target molecule may be at least two immunologically identical epitopes that are repeated on the target molecule. In which case only a single type of binding agent is required in the immunoadsorbant material. Target molecules with repetitions of epitopes are e.g. homo-multimeric protein such as e.g. immunoglobulines comprising 2 light and 2 heavy chains.

The method preferably comprises a step of selecting the binding agent. This selection is preferably done under conditions mimicking the loading and elution conditions to find a binding agent that binds with sufficient affinity to bind the target molecule on loading and which can also be eluted easily.

In one embodiment of the invention, the binding agent should have binding affinity for an epitope that occurs at least twice on the target molecule. By this selection binding agents, especially antibodies or fragments thereof, are selected, which could provide multivalent binding to a target molecule. In use two individual binding agents, especially antibody molecules, may bind to different epitopes of the same target molecule, leading to multivalent binding of the target molecule. This multivalency increases the binding affinity and the capacity of the immunoadsorbent with surprisingly high levels. Without wishing to be bound by any theory, it is believed that the multivalent binding is essential to achieve the high affinity, or rather avidity, the high dynamic binding capacity and mild elution which are objectives of the current invention.

Examples of such antibodies or fragments thereof are antibodies that specifically recognize an epitope that is present on the kappa or lambda light chain of human antibodies. In this example the human antibody is the target molecule. Human antibodies comprise two kappa light chains which comprise the epitopes.

In another embodiment of the invention in the selection step, at least two binding agents are selected, comprising one binding agent with binding affinity for an epitope (one) on the target molecule and one binding agent with binding affinity for a different epitope (two) on the target molecule. In use the adsorbent material will comprise these two different binding agents, especially antibodies or fragments thereof, that may simultaneously bind to the same target molecule, again leading to multivalent binding of the target molecule.

As mentioned above the multivalent binding of target molecule, i.e. each target molecule is bound to the immunoadsorbent material via at least two binding agent-epitope bonds, is a preferred aspect of the current invention.

It will be appreciated that the multivalent binding will take place optimally if the 3 dimensional structure of the target molecule is such that it allows this multiple binding. In practice this means that a certain distance is preferably present between the two epitopes on the target molecule. The suitability of the target molecule for multivalent binding can be derived from its 3D crystal structure.

The selection of the antibody or fragment is preferably followed by its production in larger quantities. Suitable production systems include *Saccharomyces Cerevisiae* or other yeasts, mould or bacterial expression systems.

Preferably this is followed by a step wherein the binding agent is brought into contact with immunoadsorbent material. The immunoadsorbent and the binding agent may be linked covalently or via other interactions. Preferably the binding agent is covalently coupled to an immunoadsorbent material. There are many known protocols for immobilizing proteins or fragments on adsorbent material.

It will be appreciated that where reference is made to immunoadsorbent comprising "one" or "a" binding agent, this is meant to also refer to the situation where an immunoadsorbent material is loaded with many individual binding agent molecules.

Examples of suitable immunoadsorbent material include porous solid phase carrier materials such as agarose, polystyrene, controlled pore glass, cellulose, dextrans, kieselguhr, synthetic polymers such as Sepharose™, porous amorphous silica. The carrier materials may be in any suitable format such as particles, powders, sheets, beads, filters and the like. Further specifications of suitable carrier materials are for example disclosed in EP-A-434317.

Methods are available for immobilizing ligands quickly, easily and safely through a chosen functional group. The correct choice of coupling method depends on the substance to immobilized. For example the following commercially known derivatives of Sepharose™ allow the convenient immobilization of proteins thereon: CnBr-activated Sepharose™ 4B enables ligands containing primary amino groups to be rapidly immobilized by a spontaneous reaction.

AH-Sepharose™ 4B and CH-Sepharose™ 4B both have a six-carbon long spacer arm and permit coupling via carboxyl and amino groups respectively. Flexible spacers are suitable for use in situations where the flexibility of the target molecules is limited or where 3-dimensional structure of the target requires some flexibility of the binding agent to allow optimal binding.

Activated CH-Sepharose™ 4B provides a six-carbon spacer arm and an active ester for spontaneous coupling via amino groups.

These are only a few examples of suitable immobilisation routes.

Optionally the immunoadsorbent material is put into a column to facilitate easy chromatographic separations.

In a preferred next step the immunoadsorbent material is contacted with a composition comprising the target. Often the composition will be an aqueous composition comprising many other proteins besides the target that is to be purified. The conditions of this contact step are such that binding of the binding agent, to the target molecule occurs.

Preferably in this step a loading buffer having pH around 6.5 to 8 is used. A suitable buffer is PBS buffer.

It is preferred that the loaded material is rinsed until the non specific binders have eluted.

Desorption of the target molecule is the next step. This is preferably done by changing the conditions such that the antibody or fragment no longer binds the target molecule.

Elution may be achieved by changing the conditions with respect to pH, salt, temperature or any other suitable measure. A preferred elution method for desorption is elution with a buffer having a pH below 3.

More specifically the invention relates to a method for the purification of a target molecule by immunoaffinity comprising the steps of:
a) selecting an antibody or fragment thereof, that binds to an epitope that is present at least twice on the target molecule;
b) binding the antibody or fragment thereof to immunonoadsorbent material;
c) loading the immunoadsorbent material with a composition comprising the target molecule, preferably under conditions where multivalent binding of the binding agent to the target molecule takes place;
d) washing the loaded immunoadsorbent to remove non specific binders; and,
e) eluting the target molecule by applying elution conditions.

The binding agent may be derived from any suitable source organism or may be prepared synthetically or by genetically modified organisms. It is important that the fragment retains binding affinity as defined above in the context of this invention.

Examples of suitable fragments are Fab fragments, Fv fragments.

In a preferred embodiment, the binding agents are antibodies, more preferred such antibodies or fragments thereof are derived from antibodies naturally devoid of light chains. Such antibodies are for example obtained by immunisation of llama's or sharks and purification of the antibodies thus generated. These antibodies comprise heavy chains only and are devoid of light chains. The advantage of use of these antibodies is that they are exceptionally stable even at higher temperatures, small and are easily produced in host organisms such as *Saccharomyces cerevisiae.* These antibodies are described in more detail in EP-A-656946.

More generally speaking, the binding agent preferably comprises immunoglobuline-derived variable domain that comprises a complete antigen binding site for an epitope on the target molecule in a single polypeptide chain. Such agents thus specifically include but are not limited to:
1) antibodies obtainable from camelids and sharks that consist of only heavy chains and that are naturally devoid of light chains;
2) variable domains of the antibodies defined in 1), usually referred to as VHH domains;
3) engineered forms of the antibodies defined in 1) such as e.g. "camelidised" antibodies in which frame work sequences of a camelid (or shark) VHH domain are grafted with CDRs obtained from other sources;
4) engineered forms of immunoglobuline-like variable domains in which frame works sequences from a variety of immunoglobuline-like molecules are combined with CDRs specific for a given target molecule as e.g. described in WO 04/108749.

A definition of CDR and framework sequences is given herein below. It is further understood that the frame work amino acid sequence of an immunoglobuline-derived variable domain that comprises a complete antigen binding site for an epitope on the target molecule in a single polypeptide chain preferably has at least 50, 55, 60, 65, 70, 75, 80, 85 or 90% amino acid identity with the frame work amino acid sequence of any one of SEQ ID No's 1 - 33. Preferably the immunoglobuline-derived variable domain has an affinity for the epitope as defined above herein.

A preferred binding agent is a single domain antibody fragment derived from a camelid antibody. Derived from a camelid antibody herein only means that the frame work amino acid sequences of the fragment are as defined above but that the fragment maybe designed and synthesised rather than directly obtained from or formed in a camelid organism.

We have found that the immunoadsorbent material of the invention, comprising an binding agent that binds specifically to the kappa light chain of human antibodies, show very good affinity, capacity and need only mild elution conditions.

Therefore in a specific embodiment, the invention relates to an immunoabsorbent material comprising a binding agent that has binding affinity for the kappa light chain of human antibodies.

The invention further relates to the use of such material in the purification of human antibodies.

In one embodiment the invention relates to a binding agent that is selected from the group of kappa light chain binding VHH molecules selected from SEQ ID No's 1 - 15, or a binding agent comprising an immunoglobuline-derived variable domain comprising a Complementarity Determining Region (CDR) 1, 2, and/or 3 exhibiting at least 80, 85, 90, 95, 98% amino acid identity with the CDRs of the VHH molecules of SEQ ID No's 1 - 15. Preferably, an immunoglobuline-derived variable domains have an affinity for the kappa light chain of human antibodies that is at least 10⁶ M⁻¹, 10⁷ M⁻¹, or 10⁸ M⁻¹. The CDRs 1, 2, and 3 may each individually exhibit the above defined identities or the CDRs may collectively exhibit the above defmed identities.

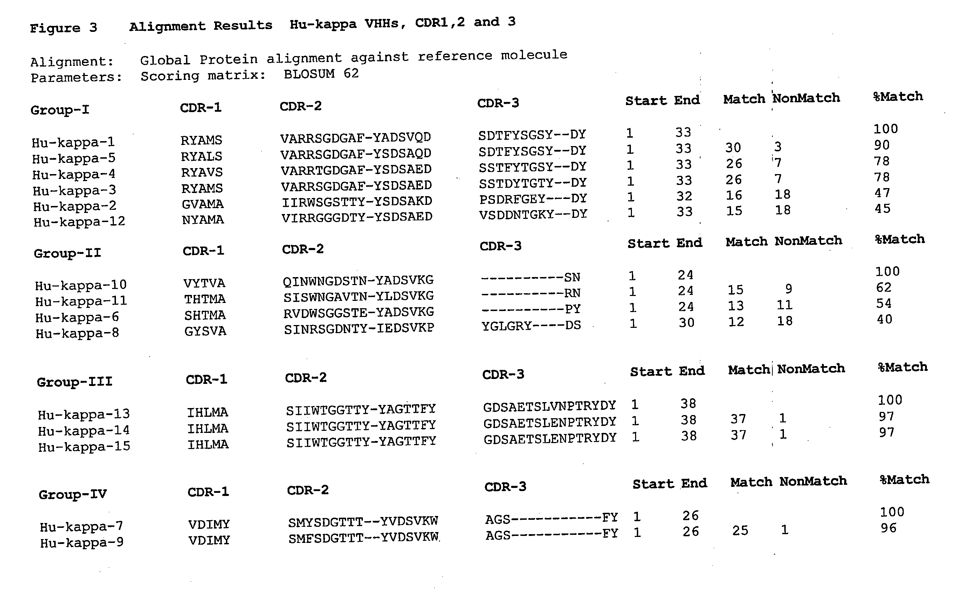

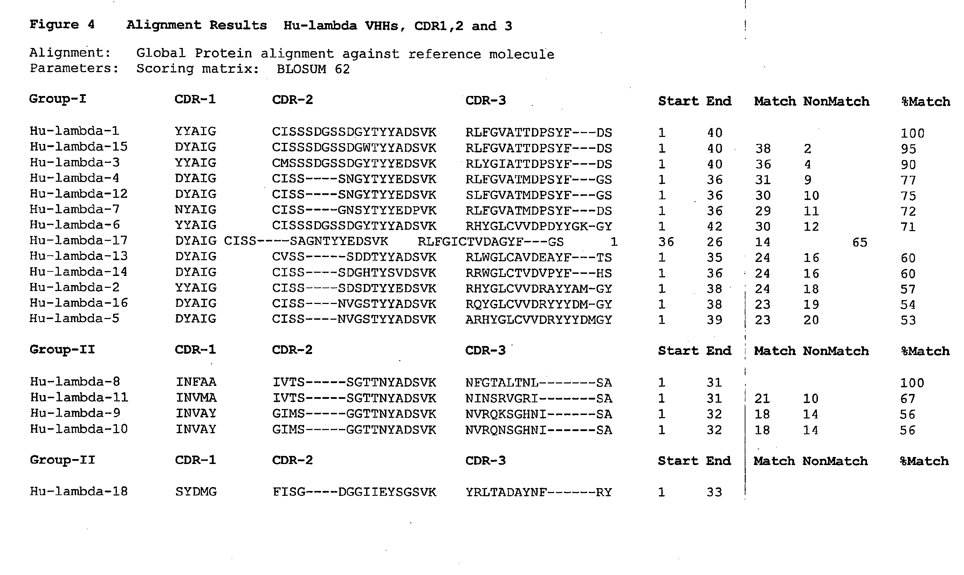

The Complementarity Determining Regions (CDR) 1, 2, and/or 3 of VHH molecules are herein defined as depicted in Figures 3 and 4. More generally, CDRs of camelid variable heavy chain domains are herein defined as described by Vu et al. (1997, Mol Immunol. 34(16-17):1121-31). The VHH sequences other than the CDR sequences in SEQ ID No's 1 - 33 or as defined by Vu et al. (1997, *supra*) are herein defmed as VHH framework sequences.

In another more specific embodiment the invention relates to an immunoadsorbent material comprising an antibody or active fragment thereof having the amino acid sequence specified below as Sequence ID 1.

In another embodiment the invention relates to an antibody or fragment having binding affinity for a human antibody, wherein the sequences comprises the sequence according to sequence ID no 1 or are essentially homologous thereto. In the context of the invention essentially homologous means that the homologous molecule shows the desired binding affinity of at least 10⁶ M⁻¹. Examples of homologous sequences are sequences that have been modified in the framework but not in the antigen binding part of the antibody according to sequence ID 1.

In yet another embodiment the invention relates to a binding agent that is selected from the group of lambda light chain binding VHH molecules selected from SEQ ID No's 16 - 33, or a binding agent comprising an immunoglobuline-derived variable domain comprising Complementarity Determining Regions (CDR) 1, 2, and/or 3 exhibiting at least 80, 85, 90, 95, 98% amino acid identity with the CDRs of the VHH molecules of SEQ ID No's 16 - 33. Preferably, an immunoglobuline-derived variable domains have an affinity for the lambda light chain of human antibodies that is at least 10⁶ M⁻¹, 10⁷ M⁻¹, or 10⁸ M⁻¹. The CDRs 1, 2, and 3 may each individually exhibit the above defmed identities or the CDRs may collectively exhibit the above defined identities.

In yet a further embodiment, the immunoadsorbant material comprises at least two different binding agents each binding an immunologically distinct epitope of a human IgG Fc domain, whereby the immunologically distinct epitopes of the human IgG Fc domain, are spatially separated as herein defined above.

In again another embodiment, the immunoadsorbant material comprises at least two different binding agents each binding an immunologically distinct epitope of human serum albumin, whereby the immunologically distinct epitopes of the albumin are spatially separated as herein defined above.

In another embodiment the current invention provides methods and immuno-adsorbent materials that are highly suitable for the adsorbtion or isolation and/or purification of immunoglobulins from crude preparations, in particular of human immunoglobulins of the types IgM, IgA, IgE and most in particular of the IgG isotypes.

Immuno adsorbent materials comprise as immunoglobulin binding agents heavy chains naturally devoid of a light chain, preferably camelid VHH's, that are specific for an epitope that is present at least twice on the target immunoglobulin, which may be identical or different targets.

In one preferred embodiment, the traget may comprise an epitope present on a light chain of the kappa or lambda isotypes, which targets are always present in duplo on an intact immunoglobulin.

In another embodiment it may be an epitope that is present twice on the Fc part of the immunoglobulin, or two different epitopes that are present on the Fc part and simultaneously accessible for interaction with the VHH binding molecule.

In yet another embodiment, the immuno adsorbent materials may comprise combinations of kappa or lambda binding antibodies with Fc binding antibodies, preferably camelid VHH antibodies, normally devoid of a light chain.

The use of immunoglobulin binding agents specific for epitopes present at least twice on the target immunoglobulin results in a surprising decrease in the dissociation rate of 10 fold to over 1000 fold in some cases, as demonstrated in the examples section for kappa binding antibodies. The decrease in the dissociation rate is useful for isolation and purification processes, as the dynamic binding capacity of immuno-adsorbent materials (or retention rate) is dramatically increased by the decreased dissociation. Secondly, the immunoglobulins can still be eluted under mild conditions, which is crucial for retaining their structural integrity and antigen binding properties.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The invention is illustrated by the following examples.

### Description of the figures

Figure 1 Alignments of human kappa and human lambda light chain binding VHHs and consensus for the CDR' regions.
Figure 2 Sensorgrams of Biacore affmity measurements; Fab - and IgG binding curves in Biacore on a VHH Hu-kappa-1 coated sensor chip (A; binding and dissociation, B; dissociation only, indicating the effect on the dissociation rate (kdiss) induced by avidity.
Figure 3 CDR amino acid sequences of human kappa light chain binding VHH molecules.
Figure 4 CDR amino acid sequences of human lambda light chain binding VHH molecules.

### Examples

### Example 1: Generation of Llama VHH ligands against kappa light chains of human antibodies.

The following protocol is taken as an example of how specific VHH fragments can be isolated, cloned, expressed, then coupled onto the desired matrix.

Although the particular VHH fragments described in this example were derived from an immune repertoire, they also could have been selected from a non-immunized VHH library (see EP1051493, Unilever) or a synthetic/semi-synthetic non-immunized VHH library (see WO00/43507, Unilever).

A llama was immunized with polyclonal IgM prepared from human serum by precipitation and gel filtration techniques and diluted in phosphate buffered saline pH 7.4 (PBS). To increase the specificity of the immune response the llama was boosted several times following the initial immunization (days 0, 28 and 49) with 250 µg of the antigen mentioned above in specol (ID-DLO, Lelystad, The Netherlands) (Boersma et al., 1992). A heparin blood sample of about 150 ml was taken 6 days after the last immunization. Peripheral blood cells were obtained via a Ficoll-Paque centrifugation. From about 2x10⁸ lymphocytes the total RNA was isolated essentially as described by Chomczynnski and Sacchi (1987). DNA encoding specific VHH fragments can then be isolated using similar methods to those described in WO 94/04678 (Casterman et al) and ligated into e.g. an episomal *Saccharomyces cerevisiae* expression vector as previously described by Frenken et.al. (2000). From these VHH expression libraries, VHH fragments with the desired antigen binding specificity (kappa or lambda light chains of human antibodies) can be selected by direct screening of culture supernatants from individual S. *cerevisiae* colonies (Frenken et.al. 2000).

Alternatively selection methods based on display techniques like phage-display and yeast display can be used to isolate anti kappa light chain VHH producing clones from immune repertoires.

For screening Nunc Maxisorp binding plates were coated with human antibody antigens and susbsequently blocked with 4% (w/v) milkpowder in PBS. Bound VHH fragments were detected by either a mouse anti-His mAb in combination with a polyclonal goat-anti-mouse-HRP conjugate (Bio-Rad, 172-1011) or a polyclonal rabbit anti-llama-VHH serum in combination with a polyclonal swine-anti-rabbit IgG-HPO conjugate (Dako, P217). Initial screening was performed on Maxisorp plates either coated with a human IgG1 monoclonal antibody that possessed a kappa light chain or a human IgG1 mab that possessed a lambda light chain. Llama VHH fragments that showed binding to IgG1 kappa only were further tested in ELISA on different human monoclonal antibodies (e.g. IgG, IgA, IgM) to confirm binding specificity towards the human kappa light chain. During the screening process, additional VHH fragments could be identified that showed binding specificity towards the human lambda light chain. The binding specificity of one of the anti human kappa VHH fragments (VHH Hu-kappa-1) as determined by ELISA is given in Table 1 and herein compared with two other VHH fragments that specifically bind to the Fc portion of human IgG antibodies (VHHs Hu-Fc-1 and Hu-Fc-2, respectively). These VHH fragments were obtained from an immune VHH library originating from a llama immunized with human Fc fragments prepared from polyclonal IgG from human serum. As demonstrated in Table 1, VHH Hu-kappa-1 recognizes an epitope that is present on kappa light chains of human antibodies and hence binds to an epitope that is present twice in the target molecule in the case of e.g. IgG, IgA and IgE antibodies or ten times in the case of IgM antibodies. Both VHH fragments Hu-Fc-1 and Hu-Fc-2 show to be specific for an epitope that is present on the Fc domain of all 4 human IgG subclasses, said epitope being present only once in the IgG target molecule.

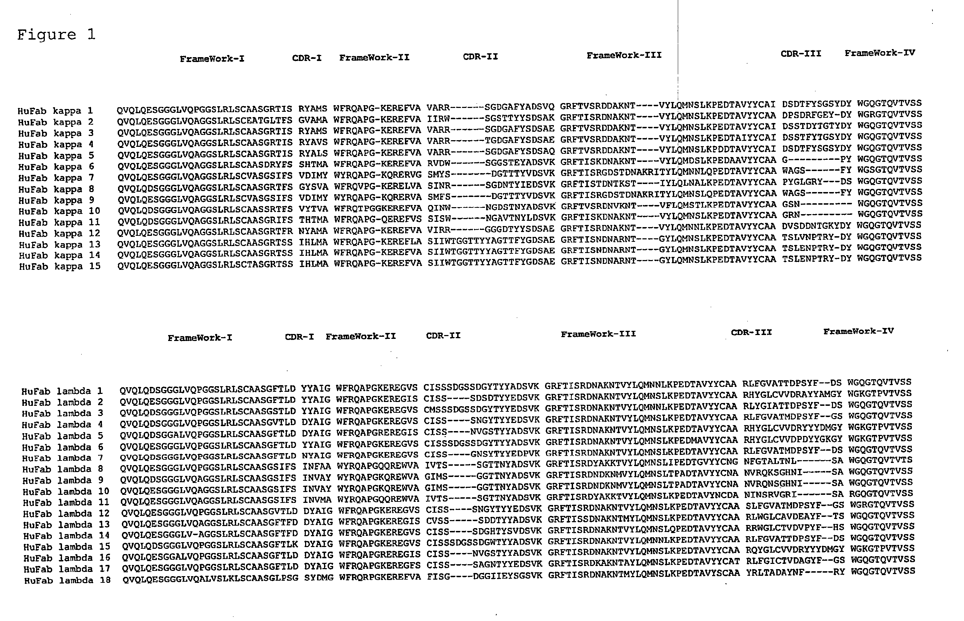

The sequence of these antibodies is presented in sequence ID no's 1 to 15 for the kappa light chain binding VHHs, no's 16 to 31 for the lambda binding VHHs. The consensus for both kappa and lambda binders is displayed in Figure 1.

**Table 1 Binding specificity of anti human kappa light chain and anti human IgG Fc VHH fragments as determined in ELISA.**

| Antibody: | | VHH fragment: | | |
|---|---|---|---|---|
| Species | Isotype / Subclass | Hu-kappa | Hu-Fc-1 | Hu-Fc-2 |
| Human | IgG₁ lambda | - | + | + |
| | IgG₂ lambda | - | + | + |
| | IgG₃ lambda | - | + | + |
| | IgG₄ lambda | - | + | + |
| | IgG₁ kappa | + | + | + |
| | IgG₂ kappa | + | + | + |
| | IgG₃ kappa | + | + | + |
| | IgG₄ kappa | + | + | + |
| | IgG, Fab fragments | + | - | - |
| | IgG, Fc fragments | - | + | + |
| | IgM (polyclonal) | + | - | - |
| | IgA (polyclonal) | + | - | - |
| Bovine | IgG (polyclonal) | - | - | - |
| Mouse | IgG (polyclonal) | - | - | - |

### Antibody production and immobilized metal affinity chromatography (IMAC) purification

The VHH antibody fragments were produced at 1-Litre or 10-Litre (shake flasks) fermentation scale using a genetically modified strain of *Saccharomyces cerevisiae* and purified using ion exchange chromatography on SP sepharose fast flow (Amersham Biosciences). The purified antibodies were dialysed against three changes of PBS buffer, pH 7.4 or the required coupling buffer, 48 hours at 4°C, using 3.5 kDa cut-off tubing (Spectra/Por 3; Spectrum Medical Industries). The concentrations of the purified samples were determined by OD₂₈₀. All purified samples were stored at -20 °C when not in use.

### Example 2 Affinity measurements

Binding affinity constants of VHH fragments Hu-kappa-1, Hu-Fc-1 and Hu-Fc-2 were determined using surface plasmon resonance analysis (SPR) on a BiaCore 3000. For this purpose, purified VHH fragments were immobilised onto the surface of a CM5 sensor chip and subsequently incubated with different concentrations of human Fab and/or human IgG antibodies in HBS-EP buffer (0.01 M HEPES, pH7,4; 0.15 M NaCl; 3 mM EDTA; 0.005% Surfactant P20). Binding was allowed for 3 minutes at 30 µl/min followed by a dissociation step of 15 minutes at 30 µl/min. Binding curves were fitted according to a 1:1 Langmuir binding model using Biacore software. An overview of the calculated affinity data is given in Table 2. With regard to the anti Hu-kappa-1 fragment the effect of avidity is clearly demonstrated by major differences in dissociation rates between Fab - and IgG molecules. Since the anti Hu-kappa-1 fragment is immobilised onto the surface of a sensor chip, said surface can interact with two identical epitopes present on one IgG molecule simultaneously. Compared to the monovalent interaction with human Fab fragments, the dissociation rate (kdiss) for IgG is significantly lower (> 1000 fold) resulting in a KD value for IgG of about 120 fM compared to 6.3 nM for Fab fragments. The latter value is in the same range as the KD values for the two anti Hu-Fc VHHs (6.4 and 2.2 nM for IgG, respectively) indicating monovalent interactions.

**Table 2 Biacore affinity data of anti Hu-kappa-1 and anti Hu-Fc VHHs (VHHs immobilised on sensor chip).**

| **VHH** | **Antigen** | **k ass** (1/Ms) | **k diss** (1/s) | **KA** (1/M) | **KD** (M) | **Avidity** |
|---|---|---|---|---|---|---|
| Hu-kappa-1 | Hu-Fab | 1.24 x 10⁵ | 7.78 x 10⁻⁴ | 1.60 x 10⁸ | 6.27 x 10⁻⁹ | **No** |
| Hu-kappa-1 | Hu-IgG | 4.18 x 10⁵ | 5.29 x 10⁻⁸ | 7. 90 x 10¹² | 1.27 x 10⁻¹³ | **Yes** |
| Hu-Fc-1 | Hu-IgG | 2.15 x 10⁵ | 1.39 x 10⁻³ | 1.55 x 10⁶ | 6.44 x 10⁻⁹ | **No** |
| Hu-Fc-2 | Hu-IgG | 8.30 x 10⁵ | 1.78 x 10⁻³ | 4.66 x 10⁸ | 2.15 x 10⁻⁹ | **No** |

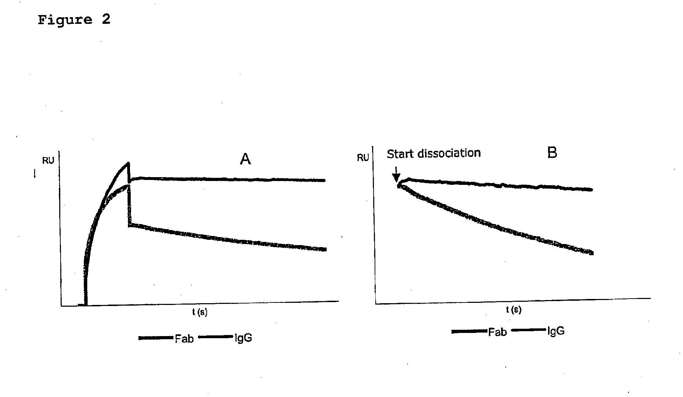

This effect on the dissociation rate (kdiss) induced by avidity is further illustrated by Figure 2, which compares Fab - and IgG Biacore binding curves (sensorgrams).

### Example 3: General materials and methods for coupling and chromatography testing Coupling to N-hydroxysuccinimide (NHS) activated sepharose 4 Fast Flow

After purification the antibodies were dialysed to NHS coupling buffer. This buffer contains 0.1 M HEPES pH 8.3. For an optimal coupling efficiency the recommended ratio of volumes coupling solution/NHS sepharose is 0.5 : 1. The antibodies had different concentrations, 0.5-15 mg/ml, the ratio of antibody/NHS sepharose of the antibodies varied between 1 : 1 and 10 : 1. When a mixture of 2 ligands is immobilised onto the matrix a 1:1 ratio of ligands was used. The following procedure was used for coupling the antibodies to NHS activated sepharose 4 Fast Flow (General Electric Healthcare). Subsequently the matrix was washed twice with NHS coupling buffer. The NHS sepharose was mixed with the antibody solution and left overnight at 4'C head over head or 1 hour at room temperature. After incubation the gel material was filtered over a sintered glass filter and the non-reacted groups of the gel material were blocked with Tris (0.1 M pH 8.0) 1 hour at room temperature. The coupled medium was washed using alternate low and high pH (3x 10 cv PBS pH 2 and 3x 10 cv PBS pH 7.4). Using the non-bound fraction the coupling efficiency was determined. This is determined by measuring the OD₂₈₀ value of the coupling solution before and after coupling. The coupling efficiency was also determined by looking at the protein pattern on SDS-PAGE of coupling solution before and after coupling.

### Chromatography experiments

Columns were made of the coupled antibody matrix using HR 5/5 columns (GE health). A column volume of 400 µl was used. All the chromatography experiments were performed on an Akta explorer 100. A two buffer system was used, buffer A1 the loading buffer was PBS pH 7.4, buffer B the elution buffer e.g. PBS with addition of 8 M HC1 to yield pH 2.1 or 0.1 M Glycine-HCl at pH 2 or 3. Different programs were used. Protein detection was performed on line by monitoring the signal of OD₂₁₄ and OD₂₈₀. Fractions were collected with a volume of 1 ml. The fractions were neutralized immediately with 20 µl Tris (2 M).

### Determination of the dynamic binding capacity of the antibody coupled matrix

To determine the dynamic binding capacity the column was loaded with the target molecule. A flowrate of 150 cm/h was used. The amount of target molecule in the flowthrough and the elution were calculated by integration of the flowthrough and elution peak area. The dynamic capacity is the elution peak area devised by the total peak area (flowthrough + elution) and this multiplied with the amount of target molecule.

### Example 4: Dynamic binding capacities of different VHH matrices for human IgG

VHH fragments (ligands) Hu-kappa-1, Hu-Fc-1 and Hu-Fc-2 were immobilised onto NHS sepharose as described earlier. Ligand densities were 20 mg of ligand per ml matrix. The dynamic binding capacity was determined using procedures as described above. The column was loaded with an amount of Human IgG higher than the expected dynamic binding capacity. Elution was performed using 0.1M Glycine buffers with pH values between 2 and 4.

As can be seen from Table 3, the highest dynamic binding capacity (DBC) for human IgG is found for the Hu-kappa-1 matrix and is almost a 2-fold higher compared to both Hu-Fc-1 - and Hu-Fc-2 matrices. Affinity measurements showed that the binding affinity (KD) of this antibody for human IgG molecules (containing 2 identical kappa light chains) is about 120 fM. This is significantly higher than the actual binding affinity of Hu-kappa-1 for its epitope present on the kappa light chains as determined with monovalent binding Fab fragments (6.3 nM). The latter figure is more in the range of both tested Human Fc specific VHH fragments and generally known compositions in the prior art. This shows that the use of immuno-adsorbent materials according to the invention leads to increased binding capacity compared to systems that do not include the feature of multivalent binding.

Another important feature of the current invention is that although multivalent binding ligands leads to high binding affinities for the target molecule, the release of said molecules can still be obtained at mild elution conditions. As demonstrated in Table 3 the optimal pH of elution for the Hu-Fc-2 matrix is lower (pH 2) compared to the Hu-kappa-1 - and the Hu-Fc-1 matrix (both pH 3). The binding strength of the Hu-Fc-2 fragment for its epitope on human IgG is almost 3-fold higher compared to Hu-Fc-1 and Hu-kappa-1 (2.2 nM versus 6.4 nM and 6.3nM, respectively). Although multivalent binding of the Hu-kappa-1 matrix increases the dynamic binding capacity for human IgG compared to e.g. the Hu-Fc-1 matrix, this avidity feature clearly does not affect conditions to obtain efficient release of bound IgG. Said conditions being comparable to those for systems that do not include the feature of multivalent binding (e.g. Hu-Fc-1 matrix).

The results as mentioned in this example are measured with a low bed height and short residence time. Higher bed heights will increase residence time and the dynamic capacity. The dynamic capacity of the described Hu-kappa-1 column at 15 cm bed height and 150cm/hr linear flow rate is 30 to 40 mg Human IgG/ml matrix. This is about a two-fold higher than the capacity of known systems that do not include the feature of multivalent binding which is specific for the current invention.

The example furthermore demonstrates that the multivalent principle of the current invention enables a unique combination of high binding capacity on one-hand and mild elution conditions on the other hand.

**Table 3 Comparison of the dynamic binding capacity (DBC) of anti Human IgG matrices.**

| Matrix | Ligand Density | DBC (mg HuIgG/ml) | pH of elution giving >90% release of bound | KD for HuIgG |
|---|---|---|---|---|
| | (mg/ml) | | IgG | (M-1) |
| Hu-kappa-1 | 20 | 24 | 3 | 1.27 x 10⁻¹³ |
| Hu-Fc-1 | 20 | 14 | 3 | 6.44 x 10⁻⁹ |
| Hu-Fc-2 | 20 | 15 | 2 | 2.15 x 10⁻⁹ |

### Example 5: Dynamic binding capacities of matrices for human IgG comprising VHHs that bind to different epitopes present in the target molecule

This example demonstrates the feature of increased binding capacity induced by multivalent binding using at least two different VHH ligands, each ligand recognizing a different epitope on the target molecule, in this case human IgG antibodies.

For this purpose two anti human IgG Fc ligands (Hu-Fc-1 and Hu-Fc-2) were used, each ligand binding to a different epitope present on the Fc domain of human IgG antibodies as demonstrated by Biacore binding analysis (see Table 4). In this Biacore experiment, purified anti human IgG Fc ligands were immobilised onto the surface of a CM5 sensor chip and subsequently incubated with human Fc fragments. This human Fc capturing step was followed by incubation with either VHH ligand Hu-Fc-1 or Hu-Fc-2. Table 4 shows that VHH ligand Hu-Fc-2 can bind to human Fc fragments when captured by immobilised VHH ligand Hu-Fc-1 and vice versa (51 RU and 181 RU, respectively) which demonstrates that each ligand binds to a different epitope present on the Fc domain of human IgG antibodies. No significant binding signals were obtained in this set-up using identical ligand pairs, indicating that each individual ligand binds to an epitope on the Fc domain of human IgG antibodies that is present or available only once.

**Table 4 Binding signals of anti Hu-Fc VHHs on captured human Fc fragments in Biacore.**

| Biacore set-up: | | | Binding Signal (RU) |
|---|---|---|---|
| Immobilized VHH | Capture target (100 µg/ml) | anti Hu-Fc VHH (100 µg/ml) | |
| Hb-Fc-1 | Human Fc | Hu-Fc-1 | 6.0 |
| Hu-Fc-1 | Human Fc | Hu-Fc-2 | 51.0 |
| Hu-Fc-2 | Human Fc | Hu-Fc-2 | 9.6 |
| Hu-Fc-2 | Human Fc | Hu-Fc-1 | 181.9 |

This further illustrates that although IgG antibodies consist of two identical heavy - and light chains, ligands directed against antibodies can recognise epitopes that are formed by a combination of identical chains such as present in the Fc domain of said antibodies. Another feature that can occur is that the ligand binds to one part of the heavy chain of the Fc domain in such a way that it prevents binding of another identical ligand due to steric hindrance.

In this respect, light chain domains of antibodies (e.g. anti human kappa light chains) as demonstrated in Example 4, have proven to be ideal epitopes to generate specific ligands against to allow multivalent binding to an epitope that is present twice in the case of antibodies. Said ligands are clearly not affected by this feature of steric hindrance.

Three different batches of anti human IgG Fc matrices were constructed (Hu-Fc-1, Hu-Fc-2 and Hu-Fc-1/2)onto NHS sepharose using methods as previously described. For each matrix the final ligand density was 2 mg of ligand per ml matrix. The dynamic binding capacity was determined using the procedure as previously described. The column was loaded with an amount of human IgG higher than the expected dynamic binding capacity. As can be seen from Table 5, the dynamic binding capacity for the mixed matrix Hu-Fc-1/2 is higher than for each individual matrix. The dynamic binding capacity for the mixed matrix (2.45 mg human IgG/ml matrix) is 1.5 times higher than the average value of the individual matrices (1.65 mg human IgG / ml matrix) that at maximum could be expected when no multivalent binding would have occurred on the mixed matrix. Based on the multivalent binding principle of the current invention these results demonstrate that high binding capacity matrices can be obtained using combinations of different ligands, each ligand binding to a different epitope present on the target molecule.

**Table 5 Comparison of the dynamic binding capacity (DBC) of mixed anti human IgG matrices.**

| Mixed Matrix | Ligand Density (mg/ml) | DBC (mg HuIgG/ml) |
|---|---|---|
| Hu-Fc-1 | 2 | 1.95 |
| Hu-Fc-2 | 2 | 1.35 |
| Hu-Fc-1 / Hu-Fc-2 | 2 | 2.45 |

### Example 6: Dynamic binding capacities of matrices for human serum albumin comprising VHHs that bind to different epitopes present in the target molecule

This example demonstrates the feature of increased binding capacity induced by multivalent binding using at least two different VHH ligands, each ligand recognizing a different epitope on the target molecule, in this case human serum albumin.

For this purpose three different anti human serum albumin (HSA) specific VHH ligands were used (HSA-1, HSA-2 and HSA-3). These VHH ligands were obtained from an immune VHH library originating from a llama immunized with human serum albumin. Biacore binding analysis according to methods as previously described revealed that ligands HSA-2 and 3 bind to the same epitope present on HSA, whereas ligand HSA-1 binds to a different epitope allowing multivalent binding of HSA when VHH ligand HSA-1 is used in combination with either VHH ligand HSA-2 or 3.

Five different batches of anti HSA matrices were constructed (HSA-1, HSA-2, HSA-3, HSA-1/2 and HSA-2/3) onto NHS sepharose using methods as previously described. For each matrix the final ligand density was 2 mg of ligand per ml matrix. The dynamic binding capacity was determined using the procedure as previously described. The column was loaded with an amount of HSA higher than the expected dynamic binding capacity. As can be seen from Table 6, the dynamic binding capacity for the mixed matrix HSA-1/2 is higher than for each individual matrix and the mixed matrix HSA-1/3. The dynamic binding capacity of mixed HSA-1/2 matrix is 2.14 mg HSA/ml matrix, which is about 1.5 times higher compared to the expected average value of 1.48 mg HSA/ml matrix when no multivalent binding would have occurred on the mixed matrix ((1.72 + 1.23)/ 2 = 1.48). The dynamic binding capacity of HSA for the mixed matrix HSA-2/3 is 0.83 mg HSA/ml matrix. This is in line with the expected average value of 0.85 mg HSA/ml matrix, since both ligands bind to the same epitope present on HSA and therefore can not induce multivalent binding leading to an increased binding capacity as demonstrated by the current invention.

**Table 6 Comparison of the dynamic binding capacity (DBC) of mixed anti HSA matrices.**

| Mixed Matrix | Ligand Density (mg/ml) | DBC (mg HSA/ml) |
|---|---|---|
| HSA-1 | 2 | 1.72 |
| HSA-2 | 2 | 1.23 |
| HSA-3 | 2 | 0.46 |
| HSA-1/2 | 2 | 2.14 |
| HSA-2/3 | 2 | 0.83 |

### References

Boersma, W.J.A., Bogaerts, W.J.C., Bianchi, A.T.J., Claassen, E., 1992. Adjuvant properties of stable water-in-oil emulsions: evaluation of the experience with specol. Res. Immunol. 143, 503-512.
Chomczynnski, P., Sacchi, N., 1987. Single step method of RNA isolation by acid guanidium thiocyanate-phenolchloroform extraction. Anal. Biochem. 162, 156-159.
Frenken G.J., Richard H.J. van der Linden, Pim W.J.J. Hermans ,J. Wil Bos a, Robin C. Ruuls, Bernard de Geus, C. Theo Verrips, 2000, Journal of Biotechnology 78, 11-21.

## Claims

1. A method for purification of an immunoglobulin, wherein:
a) the method comprises the step of binding the immunoglobulin to an immunoadsorbent material that comprises at least one mono-specific binding agent;
b) the binding agent has affinity for at least two epitopes on the immunoglobulin that are spatially separated by at least 30 angstrom; and,
c) the mono-specific binding agent is a variable domain of an antibody obtainable from a camelid that consists of only heavy chains and that is naturally devoid of light chains.

2. A method according to claim 1, wherein the avidity of the immunoadsorbant material for the immunoglobulin is at least 50 fold higher than the lowest affinity of a binding agent for an individual epitope.

3. A method according to claim 1 or 2, wherein the immunoadsorbent material comprises a first binding agent with binding affinity for a first epitope on the immunoglobulin and a second binding agent with binding affinity for a second epitope on the immunoglobulin and wherein the first and second epitopes are immunologically distinct epitopes.

4. A method according to claim 1 or 2, wherein the at least two epitopes are at least two immunologically identical epitopes that are repeated on the immunoglobulin.

5. A method according to any one of the preceding claims, wherein the binding agent comprises an immunoglobuline-derived variable domain that comprises a complete antigen binding site for an epitope on the immunoglobulin in a single polypeptide chain and whereby the framework amino acid sequences of the variable domain have at least 50% amino acid identity with the frame work amino acid sequence of any one of SEQ ID No's 1 - 33.

6. A method according to any one of the preceding claims, wherein the at least two epitopes are outside the CDR's of the immunoglobulin.

7. A method according to any one of the preceding claims, wherein at least one epitope is present on the light chain of the immunoglobulin.

8. A method according to any one of the preceding claims, wherein the at least two epitopes are epitopes of a human immunoglobulin.

9. A method according to claim 8, wherein the epitopes are epitopes of a human immunoglobulin light chain of the kappa or lambda isotype.

10. A method according to claim 9, wherein the binding agent is selected from the group of kappa light chain binding VHH molecules selected from SEQ ID No's 1 - 15, or a binding agent comprising an immunoglobuline-derived variable domain comprising a Complementarity Determining Region (CDR) 1, 2, and/or 3 exhibiting at least 80, 85, 90, 95, 98% amino acid identity with the CDRs of the VHH molecules of SEQ ID No's 1 - 15.

11. A method according to claim 9, wherein the binding agent is selected from the group of lambda light chain binding VHH molecules selected from SEQ ID No's 16 to 33, or a binding agent comprising an immunoglobuline-derived variable domain comprising a Complementarity Determining Region (CDR) 1, 2, and/or 3 exhibiting at least 80, 85, 90, 95, 98% amino acid identity with the CDRs of the VHH molecules of SEQ ID No's 16 - 33.

12. A method according to claim 8, wherein the at least two epitopes are at least two immunologically distinct epitopes of a human IgG Fc domain.

## Patentansprüche

1. Verfahren zum Reinigen von Immunglobulin, wobei:
a) das Verfahren den Schritt des Bindens des Immunglobulins an ein Immunoadsorbensmaterial aufweist, das zumindest ein monospezifisches bindendes Mittel aufweist;
b) das bindende Mittel eine Affinität für zumindest zwei Epitope am Immunglobulin aufweist, die mindestens 30 Angström räumlich getrennt sind; und
c) das monospezifische bindende Mittel eine variable Domäne eines Antikörpers ist, der von einem Schwielensohler erhalten werden kann und der nur aus schweren Ketten besteht und von Natur aus frei von leichten Ketten ist.

2. Verfahren nach Anspruch 1,
wobei die Avidität des Immunoadsorbensmaterials für das Immunglobulin mindestens 50 mal höher als die geringste Affinität eines bindenden Mittels für ein einzelnes Epitop ist.

3. Verfahren nach Anspruch 1 oder 2,
wobei das Immunoadsorbensmaterial ein erstes bindendes Mittel mit einer Bindungsaffinität für ein erstes Epitop am Immunglobulin und ein zweites bindendes Mittel mit Bindungsaffinität für ein zweites Epitop am Immunglobulin aufweist und wobei das erste und das zweite Epitop immunologisch verschiedene Epitope sind.

4. Verfahren nach Anspruch 1 oder 2,
wobei die zumindest zwei Epitope zumindest zwei immunologisch identische Epitope sind, die sich am Immunglobulin wiederholen.

5. Verfahren nach einem der vorstehenden Ansprüche,
wobei das bindende Mittel eine von Immunglobulin abgeleitete variable Domäne aufweist, die einen vollständigen Antigen-Bindungsort für ein Epitop am Immunglobulin in einer einzigen Polypeptidkette aufweist, und wobei die Aminosäuresequenzen des Gerüsts der variablen Domäne eine Identität der Aminosäuren mit der Aminosäuresequenz des Gerüsts einer der Sequenzidentifizierungsnr. 1 bis 33 von mindestens 50 % aufweisen.

6. Verfahren nach einem der vorstehenden Ansprüche,
wobei die zumindest zwei Epitope außerhalb der CDR des Immunoglobulins liegen.

7. Verfahren nach einem der vorstehenden Ansprüche,
wobei zumindest ein Epitop an der leichten Kette des Immunglobulins vorliegt.

8. Verfahren nach einem der vorstehenden Ansprüche,
wobei die zumindest zwei Epitope Epitope von Human-Immunglobulin sind.

9. Verfahren nach Anspruch 8,
wobei die Epitope Epitope einer leichten Kette des Kappa- oder Lambda-Isotyps von Human-Immunglobulin sind.

10. Verfahren nach Anspruch 9,
wobei das bindende Mittel aus der Gruppe von leichte Kappa-Ketten bindenden VHH-Molekülen, die aus den Sequenzidentifizierungsnr. 1 bis 15 ausgewählt sind, oder einem bindenden Mittel ausgewählt ist, das eine von Immunglobulin abgeleitete variable Domäne aufweist, die eine die Komplementarität bestimmende Region (CDR) 1, 2 und/oder 3 aufweist, die eine Identität der Aminosäuren mit den CDR der VHH-Moleküle der Sequenzidentifizierungsnr. 1 bis 15 von mindestens 80, 85, 90, 95, 98 % zeigt.

11. Verfahren nach Anspruch 9,
wobei das bindende Mittel aus der Gruppe von leichte Lambda-Ketten bindenden VHH-Molekülen, die aus den Sequenzidentifizierungsnr. 16 bis 33 ausgewählt sind, oder einem bindenden Mittel ausgewählt ist, das eine von Immunglobulin abgeleitete variable Domäne aufweist, die eine die Komplementarität bestimmende Region (CDR) 1, 2 und/oder 3 aufweist, die eine Identität der Aminosäuren mit den CDR der VHH-Moleküle der Sequenzidentifizierungsnr. 16 bis 33 von mindestens 80, 85, 90, 95, 98 % zeigt.

12. Verfahren nach Anspruch 8,
wobei die zumindest zwei Epitope mindestens zwei immunologisch verschiedene Epitope einer Human-IgG-Fc-Domäne sind.

## Revendications

1. Procédé de purification d'une immunoglobuline, dans lequel :
a) le procédé comprend l'étape consistant à lier l'immunoglobuline à un matériau immunoadsorbant qui comprend au moins un agent de liaison monospécifique ;
b) l'agent de liaison a une affinité pour au moins deux épitopes sur l'immunoglobuline qui sont séparés spatialement d'au moins 30 angströms ; et,
c) l'agent de liaison monospécifique est un domaine variable d'un anticorps pouvant être obtenu à partir d'un camélidé, qui est constitué uniquement de chaînes lourdes et qui est naturellement dépourvu de chaînes légères.

2. Procédé selon la revendication 1, dans lequel l'avidité du matériau immunoadsorbant pour l'immunoglobuline est au moins 50 fois plus grande que l'affinité la plus faible d'un agent de liaison pour un épitope individuel.

3. Procédé selon la revendication 1 ou 2, dans lequel le matériau immunoadsorbant comprend un premier agent de liaison ayant une affinité de liaison pour un premier épitope sur l'immunoglobuline et un deuxième agent de liaison ayant une affinité de liaison pour un deuxième épitope sur l'immunoglobuline et dans lequel les premier et deuxième épitopes sont des épitopes immunologiquement distincts.

4. Procédé selon la revendication 1 ou 2, dans lequel les épitopes, au moins au nombre de deux, sont au moins deux épitopes immunologiquement identiques qui sont répétés sur l'immunoglobuline.

5. Procédé selon l'une quelconque des revendications précédentes, dans lequel l'agent de liaison comprend un domaine variable dérivé d'une immunoglobuline qui comprend un site de liaison à l'antigène complet pour un épitope sur l'immunoglobuline dans une chaîne polypeptidique unique et moyennant quoi les séquences d'acides aminés charpentes du domaine variable ont une identité d'acides aminés d'au moins 50 % avec la séquence d'acides aminés charpentes de l'une quelconque des SEQ ID N° 1 à 33.

6. Procédé selon l'une quelconque des revendications précédentes, dans lequel les épitopes, au moins au nombre de deux, se trouvent à l'extérieur des CDR de l'immunoglobuline.

7. Procédé selon l'une quelconque des revendications précédentes, dans lequel au moins un épitope est présent sur la chaîne légère de l'immunoglobuline.

8. Procédé selon l'une quelconque des revendications précédentes, dans lequel les épitopes, au moins au nombre de deux, sont des épitopes d'une immunoglobuline humaine.

9. Procédé selon la revendication 8, dans lequel les épitopes sont des épitopes d'une chaîne légère d'immunoglobuline humaine de l'isotype kappa ou lambda.

10. Procédé selon la revendication 9, dans lequel l'agent de liaison est sélectionné dans le groupe des molécules VHH de liaison à la chaîne légère kappa sélectionnées parmi les SEQ ID n° 1 à 15, ou est un agent de liaison comprenant un domaine variable dérivé d'une immunoglobuline comprenant une région déterminant la complémentarité (CDR) 1, 2 et/ou 3 qui présente une identité d'acides aminés d'au moins 80, 85, 90, 95, 98 % avec les CDR des molécules VHH des SEQ ID N° 1 à 15.

11. Procédé selon la revendication 9, dans lequel l'agent de liaison est sélectionné dans le groupe des molécules VHH de liaison à la chaîne légère lambda sélectionnées parmi les SEQ ID n° 16 à 33, ou est un agent de liaison comprenant un domaine variable dérivé d'une immunoglobuline comprenant une région déterminant la complémentarité (CDR) 1, 2 et/ou 3 qui présente une identité d'acides aminés d'au moins 80, 85, 90, 95, 98 % avec les CDR des molécules VHH des SEQ ID N° 16 à 33.

12. Procédé selon la revendication 8, dans lequel les épitopes, au moins au nombre de deux, sont au moins deux épitopes immunologiquement distincts d'un domaine Fc d'une IgG humaine.
